# EUROPEAN PATENT SPECIFICATION

(11) **EP 0 539 470 B1**
(45) Date of publication and mention of the grant of the patent: **22.05.1996**
(21) Application number: 91913479.1
(22) Date of filing: 12.07.1991
(51) Int. Cl.: C07D 217/20, A61K 31/485

(54) **NEUROMUSCULAR BLOCKING AGENTS**
NEUROMUSKULÄRE BLOCKER
AGENTS DE BLOCAGE NEUROMUSCULAIRE

(30) Priority: 13.07.1990 GB 9015473
(43) Date of publication of application: 05.05.1993
(73) Proprietor: THE WELLCOME FOUNDATION LIMITED, London NW1 2BP (GB)
(72) Inventor: HILL, Derek Anthony, Dartford, Kent DA1 5AH (GB); TURNER, Geoffrey Lloyd, Dartford, Kent DA1 5AH (GB)
(74) Representative: Stott, Michael John
(86) International application number: GB9101150
(87) International publication number: WO9200965

(56) References cited:
- GB-A- 1 579 822
- European Journal of Medicinal Chemistry, Chimie Thérapeutique, volume 19, no. 5, septembre 1984, (Châtenay-Malabry, FR) J.B. Stenlake et al.: "Biodegradable neuromuscular blocking agents", pages 441-450, see the whole article (cited in the application)

## Description

The present invention relates to an isoquinoline compound useful as a neuromuscular blocking agent.

Neuromuscular blocking agents are widely used in surgical anaesthesia to relax the skeletal muscles to aid the work of the surgeon. Such agents are also widely used in Intensive Care Units (ICU) of hospitals to provide long-term muscle relaxation in patients who have been intubated to provide controlled ventilation of the patient.

Atracurium besylate (i.e. 2,2'-(3,11-dioxo-4,10-dioxatridecylene)-bis-(1,2,3,4-tetrahydro-6,7-dimethoxy-2-methyl-1-veratrylisoquinolinium dibesylate) is a non-depolarizing neuromuscular blocking agent which first became available for human surgical use in the UK in December 1982 and a year later in the USA. The compound is described in UK Patent Specification No. 1579822 and US Patent Specification No. 4179507. The drug is now widely used in surgery and ICU therapy. The drug was designed uniquely to undergo spontaneous degradation by "Hofmann" elimination at physiological pH and temperature and by an ester hydrolysis which proceeds independently of hepatic and renal function. For human surgical use, atracurium besylate is employed as a mixture of ten optical and geometrical isomers. Eur. J. Med. Chem-Chem, Ther. 1984-19, No. 5, pages 441-450 refer to the geometrical and optical isomers of atracurium besylate.

Neuromuscular blocking agents such as atracurium besylate act by blocking the receptor for acetylcholine at the neuromuscular junction. However, they may also block cholinergic transmission in the autonomic nervous system and produce unwanted cardiovascular side effects. For example, parasympathetic blockade results in tachycardia and hypertension whereas blockade of sympathetic ganglia will cause bradycardia and hypotension. Neuromuscular blocking agents also have the propensity of releasing histamine which can produce life-threatening anaphylactoid reactions in some patients. The antigenic group in skeletal muscle relaxant drugs is the quaternary or tertiary ammonium structure which also confers the neuromuscular blocking properties upon these agents. In this respect atracurium in a weak histamine liberator and, as with other neuromuscular blocking agents, there have been occasional reports of anaphylactoid reactions attributed to the drug.

We have now discovered that atracurium salts in which the atracurium moiety has a particular geometrical and optical isomeric configuration have an especially advantageous combination of pharmacological properties that render such salts of exceptional benefit as neuromuscular blocking agents.

The particular geometrical and optical isomeric configuration for the atracurium moiety referred to above is the 1R-cis,1'R-cis configuration. Atracurium salts having this configuration can be named as 1R-cis,1'R-cis-2',2'-(3,11-dioxo-4,10-dioxatridecylene)-bis-(1,2,3,4-tetrahydro-6,7-dimethoxy-2-methyl-1-veratrylisoquinolinium) salts. Such salts will be referred to hereinafter as 1R-cis,1'R-cis atracurium salts.

According to one feature of the present invention we provide a 1R-cis, 1'R-cis atracurium salt substantially free from other geometrical and optical isomers thereof.

The 1R-cis,1'R-cis atracurium salts according to the invention are substantially free from other geometrical and optical isomers to the extent that they are generally in admixture with less than 5% w/w, preferably less than 2% w/w of such other isomers, based on the total weight of the relevant mixture. In particular, the above 1R-cis, 1'R-cis salts according to the invention advantageously contain (a) less than 1% w/w of the corresponding cis, trans isomers and/or less than 0.5% w/w of the trans, trans isomers and/or (b) generally less than 5% w/w, preferably less than 2% w/w of the corresponding S-isomers.

For human administration, e.g. for use in surgery or medical therapy, e.g. in anaesthesia, the 1R-cis,1'R-cis atracurium salts according to the invention will include a physiologically acceptable anion, preferred anions including the halide, eg chloride, bromide or iodide, sulphate, phosphate, hydrogen phosphate, acetate, propionate, succinate, maleate and organosulphonate, eg methanesulphonate (mesylate), benzenesulphonate (besylate), p-toluenesulphonate (tosylate) and naphthalenesulphonate anions, the mesylate and besylate anions being especially preferred. Such salts containing a physiologically acceptable anion will be referred to hereinafter as physiologically acceptable 1R-cis,1'R-cis atracurium salts.

Atracurium salts including a non-physiologically acceptable anion may be employed in the synthesis of a corresponding physiologically acceptable salt.

With regard to the especially advantageous combination of pharmacological properties referred to above, we have discovered from experiments in animals that the 1R-cis,1'R-cis atracurium salts have a significantly greater neuromuscular blocking potency than atracurium besylate in the form of a mixture of geometrical and optical isomers while having a similar duration of action.

The 1R-cis,1'R-cis atracurium salts also exhibit a lower level of potential adverse effects on the autonomic nervous system including sympathetic blockade and parasympathetic blockade, and with less likelihood of producing histamine-like cardiovascular effects at therapeutic dosages, thereby providing a greater measure of patient safety, in comparison with atracurium besylate in the form of a mixture of geometrical and optical isomers.

A further advantage of the 1R-cis,1'R-cis, atracurium salts according to the invention is that they provide a more efficient neuromuscular blockade with the formation of lower levels of degradation products than the above-mentioned mixture of atracurium besylate isomers. This advantage is particularly desirable for longer surgical procedures and for ICV use involving high doses and/or long periods of treatment.

The present invention further provides:-
a) physiologically acceptable 1R-cis,1'R-cis atracurium salts according to the invention for use in surgery or medical therapy, e.g. in anaesthesia, particularly for inducing neuromuscular blockade in an animal, eg a mammal such as man;
b) the use of physiologically acceptable 1R,cis,1'R-cis atracurium salts according to the invention for the manufacture of a pharmaceutical formulation for inducing neuromuscular blockade; and
c) a method of inducing neuromuscular blockade in an animal, eg a mammal such as man which comprises administering to said animal a neuromuscular blockade - effective amount of a physiologically acceptable 1R-cis,1'R-cis atracurium salt according to the invention.

The physiologically acceptable 1R-cis,1'R-cis atracurium salts according to the invention are generally employed in surgery or medical therapy, e.g. in anaesthesia, by administering the salts to the relevant subject, e.g. man, by an appropriate route and at an appropriate dosage to achieve the desired level of neuromuscular blockade. The salts are generally administered by injection by the intravenous or intramuscular route, or, if required, by continuous intravenous infusion. The precise dosage at which the salts will be administered will vary depending on the degree of neuromuscular blockade required, the age and condition of the subject. However, when administered by the intravenous route, the salts are generally employed in a dosage of 0.1 to 0.6 mg/kg, preferably 0.2 to 0.4mg/kg. In the case of administration by infusion, the salts are generally employed in a dosage of 0.1 to 0.6 mg/kg/hour, preferably 0.2 to 0.4 mg/kg/hour.

The 1R-cis,1'R-cis atracurium salts according to the invention are generally employed in surgery or medical therapy in the form of a pharmaceutical formulation comprising such a salt together with a pharmaceutically acceptable carrier therefor. Such formulations are preferably adapted for administration by injection or infusion, eg as a solution, emulsion or suspension of the salt in a pharmaceutically acceptable aqueous or non-aqueous liquid, for example sterile water which may additionally contain if desired one or more other appropriate excipients such as bacteriostatic agents, antioxidants, buffers, thickening agents, or suspending agents. Such liquid formulations generally contain the salt in an amount of 5 to 15, preferably 5 to 10 mg/ml. Alternatively, the salts may be presented as lyophilised solids for reconstitution with water for injection or with dextrose or saline solutions. The formulations according to the invention are generally presented in unit dosage forms such as ampoules or disposable injection devices or in multidose forms such as a bottle from which the appropriate dose may be withdrawn; all such formulations should be sterile. Such unit dosage forms generally contain 10 to 250 mg preferably 25 to 50 mg of a salt according to the invention in solution or as a lyophilised solid.

The 1R-cis,1'R-cis atracurium salts according to the invention may be prepared by subjecting the corresponding 1R,1'R atracurium salt to conditions or reagents serving to effect isolation of the 1R-cis, 1'R-cis isomer from the corresponding geometrical isomers contained in the said 1R,1'R atracurium salt.

Isolation of the desired 1R-cis,1'R-cis atracurium salt in accordance with the above process is advantageously effected by chromatography, in particular high performance liquid chromatography (hplc) although liquid counter-current chromatography, column chromatography or ion exchange chromatography may also be used. Particularly efficient isolation of the desired salt has been achieved by hplc using a column packed with silica or alumina and a mobile phase comprising an appropriate mixture of solvents, e.g. a mixture of a chlorinated hydrocarbon such as methylene chloride, or acetonitrile; an alcohol e.g. a short-chain aliphatic alcohol such as methanol, ethanol or propanol; and a strong acid such as benzenesulphonic acid, methanesulphonic acid, p-toluenesulphonic acid or phosphoric acid. A mixture of methylene chloride: methanol: methane sulphonic acid, preferably in a ratio of 80:20:0.5, has been found to be especially advantageous, resulting in elution of the methanesulphonate (mesylate) salt from the column. Similarly for elution of the benzenesulphonate (besylate) salt, a solvent mixture of methylene chloride: methanol: benzenesulphonic acid (4000:500:0.25) is preferred. The eluted salt solution may subsequently be washed to remove solvents such as methanol and any excess acid and isolated by evaporation of the chlorinated hydrocarbon. The desired salt may be obtained as a solid by lyophilisation of an aqueous solution of the salt or by dissolution in a solvent such as ether then precipitation by addition of a non-polar solvent such as petroleum ether or cyclohexane.

The 1R, 1'R atracurium salt used as starting material in the above process may be prepared from (R)-1,2,3,4-tetrahydropapaverine in conventional manner, e.g. by the method described in Eur. J. Med. Chem.-Chim. Ther. 1984-19, N.,5,pages 441-450.

The following Examples illustrate the present invention:

### Example 1

a) 1,5-Pentamethylene diacrylate
   1,5-Pentanediol (15.6g) was heated in refluxing toluene (500ml) with 3-bromopropionic acid (50.5g) and a trace of p-toluenesulphonic acid for 4 hours. The cooled toluene solution was then washed with aqueous sodium acetate solution and treated with triethylamine (50ml) at reflux. The cooled reaction mixture was washed well with water to remove triethylamine and triethylamine hydrobromide and then the toluene was removed under reduced pressure.
   The product, 1,5-pentamethylene diacrylate (24.0g, 75% yield) was obtained as a pale liquid by high vacuum distillation (b.p. 90°-95°C/0.1mm Hg).
b) (R)-Tetrahydropapaverine
   (+)-Tetrahydropapaverine hydrochloride (105g) was dissolved in water and the solution was made alkaline with dilute aqueous ammonia. The precipitated (+)-tetrahydropapaverine base was dissolved in toluene and then the separated solvent was evaporaed to afford the base as a pale yellow oil. The oil was dissolved in methanol (1575ml) and treated with N-acetyl-L-leucine (47.5g). The solution was treated with diethyl ether (274ml) and (S)-tetrahydropapaverine N-acetyl-L-leucinate (35.5g) gradually crystallised out. After the crystals had been filtered off the mother liquors were evaporated to low bulk to give a solid (100g), which was then recrystallised from boiling acetone (50 volumes). Upon cooling crystals (74g, 83% (R)-diastereoisomer, 17% (S)-diastereoisomer) appeared which were filtered off. The solid was recrystallised once more from boiling acetone (50 volumes) to give 58.7g of (R)-tetrahydropapaverine N-acetyl-L-leucinate (97% (R)-isomer, 3% (S)-isomer).
c) (1R,1'R)-2,2'-(3,11-Dioxo-4,10-dioxatridecamethylene)-bis-(1,2,3,4-tetrahydro-6,7-dimethoxy-1-veratrylisoquinoline) dioxalate
   (R)-1,2,3,4-Tetrahydropapaverine N-acetyl leucinate (58.7g) was dissolved in water and treated with aqueous ammonia. The precipitated base was extracted into toluene (600mls) and, after solvent evaporation, was obtained as an oil (39.0g). The (R)-1,2,3,4-tetrahydropapaverine base was heated with 1,5-penta-methylene diacrylate (10.7g) and glacial acetic acid (3.0mls) at 70°C for 4 hours. The reaction mixture was dissolved in toluene (400ml) and stirred with silica gel 60 (Merck, column chromatography grade, 70-230 mesh), filtered and evaporated to give a yellow oil. The product was dissolved in acetone (600ml), treated with oxalic acid (9.3g) and the dioxalate salt of (1R,1'R)-2,2'-(3,11-dioxo,4,10-dioxatridecamethylene)-bis-(1,2,-3,4-tetrahydro-6,7-dimethoxy-1-veratrylisoquinoline) precipitated as a white solid (54.2g, 99% yield), m.p. 125°C, h.p.l.c. -97.8%.
d) (1R,1'R)-2,2'-(3,11-Dioxo-4,10-dioxatridecamethylene)-bis-(1,2,3,4-tetrahydro-6,7-dimethoxy-2-methyl-1-veratryliso quinolinium)benzenesulphonate ((1R,1'R) Atracurium besylate)
   (1R,1'R)-2,2'-(3,11-Dioxo-4,10-dioxatridecamethylene)-bis-(1,2,-3,4-tetrahydro-6,7-dimethoxy-1-veratrylisoquinoline)dioxalate (54.0g) was dissolved in water (1.6 litres) and treated with sodium carbonate to bring the pH to 7.0. The precipitated base was extracted into toluene (600mls) and the solvent was then evaporated to give a very viscous yellow oil (42.7g). The oil was treated with methyl benzenesulphonate (75mls) at ambient temperature overnight. The product, (1R,1'R)-2,2'-(3,11-dioxo-4,10-dioxatridecamethylene)-bis-(1,2,3,4-tetrahydro-6,7-dimethoxy-2-methyl-1-veratrylisoquinolinium)benzenesulphonate was isolated by partitioning between water and toluene. The aqueous phase was further washed with two aliquots of toluene and then subjected to lyophilisation. The product (49.7g 80% yield) was obtained as a pale yellow solid.
   The product is a mixture of (1R,1'R) atracurium besylate isomers, namely, 1R-cis,1'R-cis,1R-cis,1'R-trans and 1R-trans,1'R-trans in a ratio of 58:34:6 respectively.
e) 1R-cis,1'R-cis-2,2'-(3,11-Dioxo-4,10-dioxatridecamethylene)-bis-(1,2,3,4-tetrahydro-6,7-dimethoxy-2-methyl-1-veratryliso quinolinium)methanesulphonate ((1R-cis, 1'R-cis-Atracurium mesylate)
   A mixture of (1R,1'R)-atracurium besylate isomers (10g) obtained in stage d) was dissolved in dichloromethane (50mls) and was pumped onto an axially compressed 500mm x 50mm chromatography column packed with 520g of 20-45 micron irregular silica, and the column was eluted with a mixture of dichloromethane, methanol and methanesulphonic acid (80:20:0.5). Fractions of column eluate were collected, and the fractions containing the required 1R-cis,1'R-cis isomer were combined and washed with 10% brine. The dichloromethane solution was evaporated to dryness, the residual colourless oil was dissolved in water and the pH of the solution was adjusted to 4.0 with methanesulphonic acid. The aqueous solution was lyophilised to give the title compound (5g) as a white solid which was identified as being substantially free from other geometrical and optical isomers of the compound, namely being in admixture with less than 5% w/w of such isomers, particularly less than 3% w/w of the corresponding 1R-cis,1'S-trans isomer and less than 0.3% w/w of the corresponding 1R-cis,1'R-trans isomer.

### Example 2

### 1R-cis,1'R-cis-2,2'-(3,11-Dioxo-4,10-dioxatridecylene)-bis-(1,2,3,4-tetrahydro-6,7-dimethoxy-2-methyl-veratrylisoquino linium)benezenesulphonate ((1R-cis,1'R-cis-Atracurium besylate)

Stages a), b), c) and d) of Example 1 were repeated. The product obtained in stage d) was either isolated as described or chromatographed as described below.

A mixture of (1R,1'R)-atracurium besylate isomers (1.5g of the isomers isolated in stage d) or 3.5g of the reaction mixture from stage d)) was dissolved in dichloromethane (10mls) and was pumped onto an axially compressed 300mm x 25mm chromatography column packed with 80g of 10 micron spherical silica, and the column was eluted with a mixture of dichloromethane, methanol and benzenesulphonic acid 4000:500:0.25. Fractions of column eluate were collected, and the fractions containing the required 1R-cis,1'R-cis isomer were combined and washed with 10% brine or water. The dichloromethane solution was evaporated to dryness, the residual colourless oil or semi-solid was dissolved in water and the pH of the soltuion was adjusted to about 4.0 with an aqueous solution of benzenesulphonic acid. The aqueous solution was lyophilised to give the title compound (0.5g) as a white solid which was identified as being substantially free from other geometrical and optical isomers of the compound, namely being in admixture with less than 5% w/w of such isomers, particularly less than 3% w/w of the corresponding 1R-cis,1'S-trans isomer and less than 0.3% w/w of the corresponding 1R-cis,1'R-trans isomer. The product was analysed by ¹H NMR (CDCl₃) as follows: δ 1.52(m,7CH₂-trideca), 1.63(m,6CH₂-trideca,8CH₂-trideca), 2.84(m,1/2-4CH₂,1/2-CH₂-veratryl), 3.15(m,1/2-4CH₂), 3.22(s,NCH₃), 3.26(m,2CH₂-trideca,12CH₂-trideca), 3.34(s,OCH₃), 3.47(m,1/2-3CH₂,1/2-CH₂-veratryl), 3.58(s,OCH₃), 3.73(2s,OCH₃,OCH₃), 3.84(m,1/2-3CH₂), 3.95-4.24(m,5CH₂-trideca, 9CH₂-trideca, 1CH₂-trideca, 13CH₂-trideca), 4.86(dd,J=3,8Hz,1H), 5.87(s,8H), 6.36(dd,J=8,2Hz,6H-veratryl), 6.42(d,J=2Hz,2H-veratryl), 6.48(s,5H), 6.59(d,J=8Hz,5H-veratryl), 7.24(m,meta & para besylate), 7.78(m,ortho besylate).

The following Examples illustrate pharmaceutical formulations according to the invention in which the "Active Ingredient" is the 1R-cis, 1'R-cis-atracurium besylate salt according to the invention.

### Example 3

### Unit dose injection solution

| | | |
|---|---|---|
| Active Ingredient | | 50 mg |
| Benzenesulphonic Acid | q.s. to pH | 3 to 4 |
| Water for Injections | to make | 5 ml |

Active Ingredient is dissolved in the Water for Injections and the pH of the resulting solution is adjusted as necessary with the acid. The solution is sterilised by filtration and filled into sterile 5 ml ampoules.

### Example 4

### Multidose injection solution

| | | |
|---|---|---|
| Active Ingredient | | 100 mg |
| Benzenesulphonic Acid | q.s. to pH | 3 to 4 |
| Benzyl Alcohol | | 90 mg |
| Water for Injections | to make | 10 ml |

Active Ingredient and benzyl alcohol are dissolved in the Water for Injections and the pH of the resulting solution is adjusted as necessary with the acid. The solution is sterilised by filtration and filled into sterile 10 ml ampoules.

### Example 5

### Freeze-dried injection solution

| | | |
|---|---|---|
| Active Ingredient | | 50 mg |
| Benzenesulphonic Acid | q.s. to pH | 3 to 4 |
| Mannitol | | 62.5 mg |
| Water for Injections | to make | 2.5 ml |

The Active Ingredient and manitol are dissolved in the Water for Injections and the pH of the resulting solution is adjusted as necessary with the acid. The solution is sterilised by filtration and filled into sterile vials and freeze-dried.

### Pharmacological Evaluation

1R-cis,1'R-cis atracurium mesylate sulphonate (identified below as Compound A) was evaluated as described below, in comparison with the conventional mixture of geometrical and optical isomers (identified below as atracurium besylate) to determine the neuromuscular blocking potency and effect on sympathetic and parasympathetic blockade.
a) Methods and materials.
Mongrel male cats (Southeastern Laboratories Animal Farm), weighing 2.2-4.25kg, were anaesthetized with a mixture of pentobarbital sodium, 7mg/kg i.p., and alpha-chloralose, 80 mg/kg i.p. Adequate levels of anaesthesia were maintained with supplemental doses of alpha-chloralose administered intravenously as needed. The trachea was cannulated and the animals were ventilated with 20 ml/kg of room air via a Harvard Apparatus respiration pump adjusted to deliver 20 strokes/minute. Arterial blood pressure was measured via a cannula in the right femoral artery connected to a Statham P23 transducer. Heart rate was determined from the ECG using a Grass tachograph. The right cervical sympathetic nerve was exposed, cut approximately 5 cm proximal to the superior cervical ganglion and placed on a shielded bipolar platinum electrode. The right vagus was exposed, crushed approximately 2cm distal to the nodose ganglia, and placed on a shielded bipolar platinum electrode. The cervical sympathetic nerve and the vagus were stimulated for 10 seconds every 5 minutes with a Grass S88 stimulator using the following parameters: 20Hz, 0.5msec duration, and supramaximal voltage (10-15 volts). Isometric concentrations of the nictitating membrane were recorded during a resting tension of 5 grams with a Grass FT.03 force displacement transducer and a Grass polygraph. The resting tension on the nictitating membrane was 5 grams.
The left hind limb was rigidly secured and the tibialis tendon was isolated and attached to a Grass FT.03 force displacement transducer. After sectioning the sciatic nerve trunk, the peroneal nerve was placed on a shielded bipolar platinum electrode. Stimuli of 0.2msec duration and at a supramaximal voltage were applied to the nerve at the rate of 0.15 Hz using a Grass S88 stimulator. Twitch tension in the anterior tibialis was recorded during a resting tension of 50 grams.
The test compounds were dissolved in a buffered saline solution at a pH of 3.0 and then kept on ice and administered via a cannula in the right femoral vein. Esophageal temperature was monitored with a Yellow Springs thermistor probe and core temperature maintained between 37° and 38°C with radiant heat. All recordings were made on a Grass Model 7 polygraph. At the end of the experiments, cats were euthanatized with intravenously administered saturated KCl or pentobarbital sodium.
b) Results
The ED₉₅ value for neuromuscular blocking potency was calculated from dose-response curves.
The ED₅₀ and ED₂₅ values for the inhibitory effect of the compounds on respectively parasympathetic (vagus) and sympathetic nerve stimulation were similarly calculated.
The results are summarised in the following Table.

**TABLE**

| COMPOUND | CALC. * ED₉₅ (mg/kg) | RATIO 50% VAGUS INHIBITION vs. ED₉₅ | RATIO 25% SYMPATHETIC INHIBITION vs. ED₉₅ |
|---|---|---|---|
| Atracurium besylate | 0.092 ± 0.010 | 17 | 34 |
| Compound A | 0.062 ± 0.008 | 27 | 60 |

| | | | |
|---|---|---|---|
| * ED₉₅ calculated as mg/kg as the free base, based on the weight of the atracurium cation. | | | |

## Claims

1. A 1R-cis,1'R-cis-2,2'-(3,11-dioxo-4,10-dioxatridecamethylene)-bis-(1,2,3,4-tetrahydro-6,7-dimethoxy-2-methyl-1-veratrylisoquinolinium salt substantially free from other geometrical and optical isomers thereof.

2. A 1R-cis,1'R-cis-2,2'-(3,11-dioxo-4,10-dioxatridecamethylene)-bis-(1,2,3,4-tetrahydro-6,7-dimethoxy-2-methyl-1-veratrylisoquinolinium salt as claimed in claim 1 in admixture with less than 5% w/w of other geometrical and optical isomers thereof.

3. A 1R-cis,1'R-cis-2,2'-(3,11-dioxo-4,10-dioxatridecamethylene)-bis-(1,2,3,4-tetrahydro-6,7-dimethoxy-2-methyl-1-veratrylisoquinolinium salt as claimed in claim 2 in admixture with less than 2% w/w of other geometrical and optical isomers thereof.

4. A physiologically acceptable 1R-cis,1'R-cis-2,2'-(3,11-dioxo-4,10-dioxatridecamethylene)-bis-(1,2,3,4-tetrahydro-6,7-dimethoxy-2-methyl-1-veratryliso-quinolinium salt as claimed in any of the preceding claims.

5. The 1R-cis,1'R-cis-2,2'-(3,11-dioxo-4,10-dioxatridecamethylene)-bis-(1,2,3,4-tetrahydro-6,7-dimethoxy-2-methyl-1-veratrylisoquinolinium mesylate or besylate salt as claimed in claim 4.

6. A physiologically acceptable 1R-cis,1'R-cis-2,2'-(3,11-dioxo-4,10-dioxatridecamethylene)-bis-(1,2,3,4-tetrahydro-6,7-dimethoxy-2-methyl-1-veratryliso-quinolinium salt as claimed in claim 4 for use in surgery or medical therapy.

7. A physiologically acceptable 1R-cis,1'R-cis-2,2'-(3,11-dioxo-4,10-dioxatridecamethylene)-bis-(1,2,3,4-tetrahydro-6,7-dimethoxy-2-methyl-1-veratryliso-quinolinium salt as claimed in claim 6 for inducing neuromuscular blockade in an animal.

8. The use of a physiologically acceptable 1R-cis,1'R-cis-2,2'-(3,11-dioxo-4,10-dioxatridecamethylene)-bis-(1,2,3,4-tetrahydro-6,7- dimethoxy-2-methyl-1-veratryliso-quinolinium salt as claimed in either of claims 4 and 5 for the manufacture of a medicament for inducing neuromuscular blockade.

9. A pharmaceutical formulation comprising a physiologically acceptable 1R-cis,1'R-cis-2,2'-(3,11-dioxo-4,10-dioxatridecamethylene)-bis-(1,2,3,4-tetrahydro-6,7-dimethoxy-2-methyl-1-veratryliso-quinolinium salt as claimed in either of claims 4 and 5 together with a pharmaceutically acceptable carrier therefor.

10. A pharmaceutical formulation as claimed in claim 9 adapted for administration by injection or infusion.

11. A process for the preparation of a 1R-cis,1'R-cis-2,2'-(3,11-dioxo-4,10-dioxatrideca-methylene)-bis-(1,2,3,4-tetrahydro-6,7-dimethoxy-2-methyl-1-veratryliso-quinolinium salt as claimed in claim 1 which comprises subjecting a corresponding 1R,1'R atracurium salt to conditions or reagents serving to effect isolation of the 1R-cis,1'R-cis isomer from the corresponding geometrical isomers contained in the said 1R,1'R atracurium salt.

## Patentansprüche

1. 1R-cis,1'R-cis-2,2'-(3,11-dioxo-4,10-dioxatridecamethylen)-bis-(1,2,3,4-tetrahydro-6,7-dimethoxy-2-methyl-1-veratrylisochinolinium)salz, das von anderen geometrischen und optischen Isomeren davon, im wesentlichen frei ist.

2. 1R-cis,1'R-cis-2,2'-(3,11-dioxo-4,10-dioxatridecamethylen)-bis-(1,2,3,4-tetrahydro-6,7-dimethoxy-2-methyl-1-veratrylisochinolinium)salz nach Anspruch 1 im Gemisch mit weniger als 5 % Gew./Gew. von anderen geometrischen und optischen Isomeren davon.

3. 1R-cis,1'R-cis-2,2'-(3,11-dioxo-4,10-dioxatridecamethylen)-bis-(1,2,3,4-tetrahydro-6,7-dimethoxy-2-methyl-1-veratrylisochinolinium)salz nach Anspruch 2 im Gemisch mit weniger als 2 % Gew./Gew. von anderen geometrischen und optischen Isomeren davon.

4. Physiologisch annehmbares 1R-cis,1'R-cis-2,2'-(3,11-dioxo-4,10-dioxatridecamethylen)-bis-(1,2,3,4-tetrahydro-6,7-dimethoxy-2-methyl-1-veratrylisochinolinium)salz nach einem der vorhergehenden Ansprüche.

5. 1R-cis,1'R-cis-2,2'-(3,11-dioxo-4,10-dioxatridecamethylen)-bis-(1,2,3,4-tetrahydro-6,7-dimethoxy-2-methyl-1-veratrylisochinolinium)mesylat- oder -besylatsalz nach Anspruch 4.

6. Physiologisch annehmbares 1R-cis,1'R-cis-2,2'-(3,11-dioxo-4,10-dioxatridecamethylen)-bis-(1,2,3,4-tetrahydro-6,7-dimethoxy-2-methyl-1-veratrylisochinolinium)salz nach Anspruch 4 zur Verwendung in der Chirurgie oder der medizinischen Therapie.

7. Physiologisch annehmbares 1R-cis,1'R-cis-2,2'-(3,11-dioxo-4,10-dioxatridecamethylen)-bis-(1,2,3,4-tetrahydro-6,7-dimethoxy-2-methyl-1-veratrylisochinolinium)salz nach Anspruch 6 zur Induzierung einer neuromuskulären Blockade in einem Tier.

8. Verwendung eines physiologisch annehmbaren 1R-cis,1'R-cis-2,2'-(3,11-dioxo-4,10-dioxatridecamethylen)-bis-(1,2,3,4-tetrahydro-6,7-dimethoxy-2-methyl-1-veratrylisochinolinium)salzes nach einem der Ansprüche 4 und 5 zur Herstellung eines Arzneimittels zur Induzierung einer neuromuskulären Blockade.

9. Pharmazeutisches Präparat, enthaltend ein physiologisch annehmbares 1R-cis,1'R-cis-2,2'-(3,11-dioxo-4,10-dioxatridecamethylen)-bis-(1,2,3,4-tetrahydro-6,7-dimethoxy-2-methyl-1-veratrylisochinolinium)salz nach einem der Ansprüche 4 und 5 zusammen mit einem pharmazeutisch annehmbaren Träger hierfür.

10. Pharmazeutisches Präparat nach Anspruch 9, das für die Verabreichung durch Injektion der Infusion angepaßt ist.

11. Verfahren zur Herstellung eines 1R-cis,1'R-cis-2,2'-(3,11-dioxo-4,10-dioxatridecamethylen)-bis-(1,2,3,4-tetrahydro-6,7-dimethoxy-2-methyl-1-veratrylisochinolinium)salzes nach Anspruch 1, umfassend die Unterwerfung eines entsprechenden lR,l'R-Atracuriumsalzes, Bedingungen oder Reagentien, die dazu dienen, eine Isolierung des entsprechenden 1R-cis,1'R-cis-Isomeren aus den entsprechenden geometrischen Isomeren, die in dem genannten 1R,1'R-Atracuriumsalz enthalten sind, zu bewirken.

## Revendications

1. Sel de 1R-cis,1'R-cis-2,2'-(3,11-dioxo-4,10-dioxatridécaméthylène)bis-(1,2,3,4-tétrahydro-6,7-diméthoxy-2-méthyl-1-vératrylisoquinoléinium sensiblement dépourvu d'autres isomères géométriques et optiques de celui-ci.

2. Sel de 1R-cis,1'R-cis-2,2'-(3,11-dioxo-4,10-dioxatridécaméthylène)bis-(1,2,3,4-tétrahydro-6,7-diméthoxy-2-méthyl-1-vératrylisoquinoléinium suivant la revendication 1, en mélange à moins de 5% p/p d'isomères géométriques et optiques de celui-ci.

3. Sel de 1R-cis,1'R-cis-2,2'-(3,11-dioxo-4,10-dioxatridécaméthylène)bis-(1,2,3,4-tétrahydro-6,7-diméthoxy-2-méthyl-1-vératrylisoquinoléinium suivant la revendication 2, en mélange à moins de 2% p/p d'isomères géométriques et optiques de celui-ci.

4. Sel de 1R-cis,1'R-cis-2,2'-(3,11-dioxo-4,10-dioxatridécaméthylène)bis-(1,2,3,4-tétrahydro-6,7-diméthoxy-2-méthyl-1-vératrylisoquinoléinium physiologiquement acceptable, suivant l'une quelconque des revendications précédentes.

5. Mésylate sel ou bésylate sel de 1R-cis,1'R-cis-2,2'-(3,11-dioxo-4,10-dioxatridécaméthyléne)bis-(1,2,3,4-tétrahydro-6,7-diméthoxy-2-méthyl-1-vératrylisoquinoléinium suivant la revendication 4.

6. Sel de 1R-cis,1'R-cis-2,2'-(3,11-dioxo-4,10-dioxatridécaméthylène)bis-(1,2,3,4-tétrahydro-6,7-diméthoxy-2-méthyl-1-vératrylisoquinoléiniumphysiologiquement acceptable, suivant la revendication 4, en vue de son utilisation dans le domaine chirurgical ou thérapeutique médical.

7. Sel de 1R-cis,1'R-cis-2,2'-(3,11-dioxo-4,10-dioxatridécaméthylène)bis-(1,2,3,4-tétrahydro-6,7-diméthoxy-2-méthyl-1-vératrylisoquinoléinium physiologiquement acceptable, suivant la revendication 6, en vue d'induire un blocage neuromusculaire dans un animal.

8. Utilisation d'un sel de 1R-cis,1'R-cis-2,2'-(3,11-dioxo-4,10-dioxatridécaméthylène)bis-(1,2,3,4-tétrahydro-6,7-diméthoxy-2-méthyl-1-vératrylisoquinoléinium physiologiquement acceptable, suivant l'une quelconque des revendications 4 et 5, en vue de la fabrication d'un médicament destiné à induire un blocage neuromusculaire.

9. Composition pharmaceutique comprenant un sel de 1R-cis,1'R-cis-2,2'-(3,11-dioxo-4,10-dioxatridécaméthylène)bis-(1,2,3,4-tétrahydro-6,7-diméthoxy-2-méthyl-1-vératrylisoquinoléinium physiologiquement acceptable, suivant l'une quelconque des revendications 4 et 5, ainsi qu'un véhicule ou excipient pharmaceutiquement acceptable pour ce composé.

10. Composition pharmaceutique suivant la revendication 9, adaptée à l'administration par injection ou par perfusion.

11. Procédé de préparation d'un sel de 1R-cis,1'R-cis-2,2'-(3,11-dioxo-4,10-dioxatridécaméthyléne)bis-(1,2,3,4-tétrahydro-6,7-diméthoxy-2-méthyl-1-vératrylisoquinoléinium suivant la revendication 1, caractérisé en ce que l'on soumet un sel de 1R,1'R atracurium correspondant à des conditions ou à l'action de réactifs servant à réaliser l'isolement de l'isomère 1R-cis,1'R-cis à partir des isomères géométriques correspondants contenus dans ledit sel de 1R,1'R atracurium.
